# EUROPÄISCHE PATENTSCHRIFT

(11) **EP 2 885 035 B1**
(45) Veröffentlichungstag und Bekanntmachung des Hinweises auf die Patenterteilung: **02.05.2018**
(21) Anmeldenummer: 13794962.4
(22) Anmeldetag: 20.08.2013
(51) Int. Cl.: A61M 5/36, A61M 39/24, A61M 39/26, F16K 27/02, F16K 15/02

(54) **EINWEG-VENTILEINRICHTUNG**
DISPOSABLE VALVE DEVICE
SYSTÈME DE VALVE UNIDIRECTIONNELLE

(30) Priorität: 20.08.2012 DE 202012007884 U
(43) Veröffentlichungstag der Anmeldung: 24.06.2015
(73) Patentinhaber: Illinois Tool Works Inc., Glenview, IL 60025 (US)
(72) Erfinder: MIJERS, Willem, Marinus, Mijers, 2014 Haarlem, AL North Holland (NL)
(74) Vertreter: Weickmann & Weickmann PartmbB
(86) Internationale Anmeldenummer: PCT/IB2013/001805
(87) Internationale Veröffentlichungsnummer: WO 2014/072774

(56) Entgegenhaltungen:
- EP-A1- 0 648 513
- WO-A1-03/092788
- DE-C1- 19 749 562
- GB-A- 2 443 260
- US-A1- 2010 211 020

## Beschreibung

Die Erfindung betrifft eine Einweg-Ventileinrichtung, insbesondere ein Niederdruck-Rückschlagventil zu Verwendung für ein Infusionsbesteck vorzugsweise für Schwerkraft- und Pumpeninfusion, bestehend aus einem Gehäuse mit einem Zuleitungs-und einem Ableitungsrohr und mit zumindest einer in einem Druckraum einliegenden Dichtung.

Ventileinrichtungen der gattungsgemäßen Art zur Erzeugung eines Leerlaufstops werden bevorzugt für Infusionsbestecke in Krankenhäusern eingesetzt, wobei die Ventileinrichtungen dafür verwendet werden, dass beim Leerlaufen eines mit Infusionsflüssigkeit gefüllten Vorratsbehälters ein sofortiger Leerlaufstop eintritt, damit keine Luft in die Infusionsflüssigkeit und gegen Ende des Zustroms über die vorhandenen Leitungen in das Venensystem eines Patienten gelangt. Eine Ventileinrichtung zur Verwendung in einem Infusionsbesteck ist beispielsweise aus der deutschen Offenlegungsschrift DE 29 19 343 A1 bekannt, welches mit einer Tropfkammer und einem Schwimmerventil ausgestattet ist, das bei leerlaufender Kammer das Eintreten von Luft in den Infusionsschlauch verhindert. Eine weitere Leerlaufsicherung ist aus der deutschen Offenlegungsschrift DE 36 32 412 A1 bekannt, bei der vor einer Tropfkammer ein Leerlaufventil in Strömungsrichtung angeordnet ist.

Aus der DE 197 49 562 A1 ist ferner ein Infusionsbesteck bekannt, das aus einem hochaufgehängten Behälter für die Infusionsflüssigkeit sowie einem an einem Drosselbehälter mittels eines Dornrohres anschließbaren Tropf, einer darunter auf einer flexiblen Zuleitung einstellbaren Rollenklemme und einer am Leitungsende befindlichen Injektionsnadel besteht. Um das Eindringen von Luft in die Infusionsflüssigkeit, insbesondere gegen Ende des Zustroms der Infusionsflüssigkeit zu verhindern, wird in Strömungsrichtung vor der Tropfkammer oder an deren Eingangsöffnung ein Leerlaufstop in die Strömung vorgeschaltet, mittels dessen eingebautem Ventils abhängig von einem anstehenden statischen Druck der Durchströmung die Infusionsflüssigkeit abstoppbar ist. Im praktischen Einsatz hat sich jedoch gezeigt, dass ein Leerlaufstop in Abhängigkeit des statisch anstehenden Drucks der Infusionsflüssigkeit zur Verhinderung des Eindringens von Luft nicht ausreichend ist, insbesondere dann, wenn die Möglichkeit eines Vertauschens der Anschlüsse durch Unachtsamkeit gegeben und somit die Funktion des Leerlaufstop-Ventils nicht mehr gewährleistet ist.

Dies wird besonders gefährlich, wenn Luft in das System eindringt, die beim Patienten, falls die Luft bis in die Venen vordringt, zu einer Embolie führen kann. Es ist daher wichtig, dass zusammen mit der Infusionsflüssigkeit keine Luft mitgeführt wird, die zu dem vorgenannten Nachteil führt. Ein besonderer Nachteil besteht darin, dass das vorbekannte Ventil im Falle des Anstehens eines statischen Überdrucks ausgangsseitig das Eindringen von Luft in das System nicht verhindern kann.

EP-A1-0648513 betrifft die Vorrichtungen zum medizinischen Gebrauch für die Injektion von Flüssigkeiten, bestehend aus Leitungsmittel zur Hindurchleitung von Flüssigkeiten und einem Rückschlagschwellenventil zum Hindurchleiten von Flüssigkeiten in einer Richtung zwischen dem Ausgang einer Druckflüssigkeits-Versorgungsrichtung und dem Eingang einer Injektionsleitung.

GB-A-2443260 betrifft eine Einweg-Ventileinrichtung, in welcher ein Membran als eine Dichtung verwendet wird.

Ferner offenbart US-AL-2010/211020 eine nadelfreie Vorrichtung.

Schließlich ist aus der DE 102 19 994 A1 bzw. aus der WO 031092788 A1 eine Einweg-Ventileinrichtung bekannt, die an den Nachteilen des vorgenannten Standes der Technik ansetzt und eine Ventileinrichtung vorschlägt, bei der das Eindringen von Luft in das System des Infusionsbestecks mit höherer Sicherheit verhindert werden kann. Hierfür ist vorgesehen, dass durch einen an dem Zuleitungs- und/oder Ableitungsrohr anstehenden statischen Unterdruck oder einen Überdruck an dem Ableitungsrohr eine Unterbrechung des Durchflusses erfolgt. Zur Abdichtung des Zuleitungs- und Ableitungsrohres ist hierbei eine Dichtung vorgesehen, welche innerhalb eines Druckraumes auf einer Seite an einer ersten Dichtringlippe und auf der gegenüberliegenden Seite an einer zweiten Dichtringlippe wechselseitig jeweils unter Spannung anliegt. Mittels der dadurch realisierten Dichtungen erfolgt eine Abdichtung der Dichtringlippen, welche in Wirkverbindung mit dem Zuleitungs- bzw. Ableitungsrohr stehen und im Falle des Anliegens eines Unterdrucks eine Abdichtung gegenüber den Dichtringlippen ermöglichen. Aufgrund der wechselseitigen Abdichtung der Dichtringlippen durch zumindest eine Dichtung ist sichergestellt, dass im Falle eines Überdrucks an dem Ableitungsrohr nur eine Anhebung der Dichtung an der dem Ableitungsrohr zugeordneten Dichtringlippe stattfindet, während hingegen die Dichtung auf die zweite Dichtringlippe aufgrund des vorherrschenden Überdrucks gepresst wird und somit ein Verschluss des Zuleitungsrohrs erfolgt. Alternativ zum Einsatz eines einzigen Druckraumes mit einer darin befindlichen Dichtung, das heißt einer einzigen Membran, besteht auch die Möglichkeit, dass zwei über einen Verbindungskanal kommunizierende Druckkammern ausgebildet sind, und eine zweiteilige Dichtung verwendet wird.

Nachteilig an der vorgeschlagenen Ventileinrichtung ist, dass der Gesamtaufbau eine unangemessene Größe erfordert, was insbesondere bei Kombination der Ventileinrichtung mit einer Injektionspumpe ein so erhebliches Ausmaß annehmen kann, dass ein versehentliches Abbrechen der Gesamtvorrichtung als Risiko besteht.

Aufgrund der großen Abmessungen sind auch die Kosten der Ventileinrichtung relativ hoch. Auch die Kosten der speziellen Gummimembran sind relativ hoch, wobei die Membran bzw. die gesamte Ventileinrichtung auf Grund ihrer Komplexität nur schlecht in einem automatisierten Herstellungsverfahren produziert werden kann.

Zudem besteht der Nachteil, dass der Öffnungsdruck der Ventileinrichtung relativ hoch ist und vorrichtungsbedingt nicht verringert werden kann. Der Öffnungsdruck liegt bei etwa dem Druck einer 50 cm-Wassersäule. Demnach ist der Einsatz der Ventileinrichtung nur in Kombination mit Infusionspumpen geeignet, jedoch nicht bei solchen, die mit Gravitationsdruck arbeiten.

Der Erfindung liegt daher die Aufgabe zugrunde, eine verbesserte Einweg-Ventileinrichtung aufzuzeigen, welche die vorgenannten Nachteile aus dem Stand der Technik der DE 102 19 99 A1 behebt.

Erfindungsgemäß ist zur Lösung der Aufgabe gemäß Anspruch 1 vorgesehen, dass - im Gegensatz zu der in der DE 102 19 994 A1 vorgeschlagenen Ventileinrichtung - nicht zwei Druckkammern, die zur jeweiligen Abdichtung dasselbe Membran verwenden, in derselben Ebene angeordnet werden, sondern dass zwei Druckkammern in Verlaufsrichtung des zu verabreichenden Mittels hintereinander angeordnet werden. Dies bedeutet, dass die beiden Druckkammern mit ihren Grundflächen übereinander liegen. Hieraus ergibt sich der Vorteil, dass die Ventileinrichtung deutlich kleinere Abmessungen aufweisen kann, insbesondere bezüglich ihrer in Relation zur Verlaufsrichtung gesehenen Breite. Die beiden Druckkammern liegen also nicht in derselben Ebene, sondern in zwei unterschiedlichen, die vorzugsweise parallel zueinander sind. Dabei ist es wünschenswert, dass die beiden Druckkammern sich in einer Aufsicht zumindest teilweise überlappen. Mit Aufsicht ist die Verlaufsrichtung gemeint bzw. die Richtung von der einen Grundseite des Zylinders des Gehäuses zur anderen Seite bzw. von der ersten Seite des Gehäuses zu seiner zweiten Seite.

Beispielsweise kann die Ventileinrichtung dadurch eine zylindrische Form aufweisen, wobei die erste Druckkammer im oberen Bereich des Zylinders angeordnet ist (wenn der Zylinder auf einer Stirnfläche steht) und die zweite Druckkammer im unteren Bereich.

Durch das Hintereinander-Schalten der beiden Druckkammern kann auch der Öffnungsdruck deutlich besser eingestellt werden und geringere Werte erreichen, so dass beispielsweise ein Infusionsbesteck mit Gravitationsdruck in Kombination mit der vorgeschlagenen Ventileinrichtung verwendet werden kann.

Zudem besteht noch der Vorteil, dass die vorgeschlagene Ventileinrichtung einfacher mit nadelfreien Ventilen verwendbar ist, wie sie derzeit auf dem Markt angeboten werden.

Es sind zwar im Vergleich zum Stand der Technik mehr Komponenten notwendig, jedoch können diese leichter hergestellt und zusammengebaut werden, so dass die Produktionskosten insgesamt reduziert werden können. Zudem kann die benötigte Zeit zur Herstellung verringert werden, da insgesamt die Komponenten eine geringere Komplexität aufweisen.

Bei der erfindungsgemäßen Ventileinrichtung ist es notwendig, dass mindestens zwei Dichtungen oder Membranen verwendet werden. Diese sind erfindungsgemäß ebenfalls in Bezug zur Verlaufsrichtung des zu verabreichenden Mittels hintereinander geschaltet. Die beiden Dichtungen oder Membrane liegen also nicht in derselben Ebene, sondern in zwei verschiedenen, die vorzugsweise parallel zueinander sind.

Dabei ist es wünschenswert, dass sich die beiden Dichtungen in der Aufsicht zumindest teilweise überschneiden.

Von der Funktionsweise entspricht die erfindungsgemäße Ventileinrichtung ansonsten jedoch der aus DE 102 19 994 A1 bekannten.

Weiterhin wird vorgeschlagen, dass das erfindungsgemäße Gehäuse der Ventileinrichtung ein Hauptteil und einen Gehäuseaufsatz aufweist. Diese beiden Teile sollten dicht miteinander verschlossen sein. Dies lässt sich beispielsweise mittels Ultraschall-Schweißen bei der Verbindung der beiden Teile miteinander realisieren. Vorzugsweise weist der Gehäuseaufsatz eine Zuleitung in die Ventileinrichtung und der Hauptteil eine Ableitung aus der Ventileinrichtung für das zu verabreichende Mittel auf. Eine erste Dichtringlippe steht dabei mit der Zuleitung in Wirkverbindung und eine zweite Dichtringlippe steht mit der Ableitung in Wirkverbindung.

Die zusammengebauten Teile des Gehäuses bilden vorzugsweise einen Hohlraum, in dem die Dichtungen oder Membranen angeordnet sind. Zudem kann in dem Hohlraum, vorzugsweise zwischen den beiden Dichtungen oder Membranen, ein inneres Ventifteil angeordnet sein, welches zur Befestigung der Dichtungen oder Membranen und/oder zur Abdichtung gemeinsam mit den Dichtungen oder Membranen und/oder zur Leitung des zu verabreichenden Mittels durch die Ventileinrichtung hindurch dienen kann.

In der vorgenannten Aussparung zwischen dem Hauptteil und dem Gehäuseaufsatz des Gehäuses sind auch die erste und die zweite Druckkammer angeordnet. Die Wände der ersten und der zweiten Druckkammer werden also durch das innere Teil, die erste und/oder die zweite Dichtung definiert, und können vorzugsweise noch durch das Hauptteil oder den Gehäuseaufsatz definiert werden.

Die erste Dichtung ist erfindungsgemäß Teil einer ersten Ventilscheibe und die zweite Dichtung ist erfindungsgemäß Teil einer zweiten Ventilscheibe. Die Ventilscheiben und/oder die Dichtungen oder Membranen können Durchgangslöcher aufweisen.

Dabei ist die erste Ventilscheibe zwischen dem inneren Gehäuseteil und dem Gehäuseaufsatz derart eingeklemmt, dass die Dichtung oder Membran unter Vorspannung gegen den Gehäuseaufsatz anliegt. Alternativ kann die Dichtung oder Membran auch gegen die Ventilscheibe unter Vorspannung anliegen.

Die zweite Ventilscheibe ist zwischen dem inneren Gehäuseteil und dem Hauptteil derart eingeklemmt, dass die Dichtung oder Membran unter Vorspannung gegen die Innenseite der zweiten Ventilscheibe und/oder gegen das Hauptteil anliegt.

Dadurch kann bewirkt werden, dass dieselben technischen Effekte wie beim Stand der Technik DE 102 19 994 A1 erzielt werden, nämlich dass durch einen an der Zuleitung und/oder der Ableitung anstehenden statischen Unterdruck oder einen Überdruck an der Ableitung eine Unterbrechung des Durchflusses erfolgt. Ein statischer Unterdruck an dem Zuleitungsrohr kann beispielsweise dann entstehen, wenn ein Vorratsbehälter der Infusionsflüssigkeit bzw. des zu verabreichenden Mittels leergelaufen ist, wenn eine Infusionspumpe fehlerhaft arbeitet oder Luft in das System eingetreten ist. Ein Überdruck an dem Ableitungsrohr kann beispielsweise bei einem vorübergehenden Verschluss der Armvenen des Patienten auftreten, wenn ausgangsseitig eine Medikamentenpumpe zusätzlich angeschlossen ist. Ein Überdruck würde dazu führen, dass das Medikament in das Schwerkraft-Infusionsbesteck gepumpt wird.

Durch die erfindungsgemäße Ventileinrichtung wird jedoch sichergestellt, dass in den vorgenannten Fällen ein weiterer Transport der Infusionsflüssigkeit zuverlässig gestoppt wird und somit das Eindringen von mit Luft angereicherter Infusionsflüssigkeit in die Venen verhindert wird. Zusätzlich wird durch das Einwege-Ventil sichergestellt, dass ausgangsseitig bei anstehenden Überdruck durch eine Infusions- oder Medikamentenpumpe das Ventil schließt und somit das Medikament nicht in das Infusionsbesteck gelangt, wodurch eine kurzzeitige Überdosierung verhindert wird.

Vorzugsweise werden zur Abdichtung zusätzlich zu den Dichtungen oder Membranen Dichtringlippen oder Dichtlippen verwendet. Diese können dabei auf den Teilen des Gehäuses angeordnet sein, das heißt beispielsweise auf dem Gehäuseaufsatz und/oder auf dem Hauptteil. Dabei liegen die Dichtungen oder Membrane vorzugsweise unter Vorspannung gegen die Dichtringlippen an.

Alternativ ist denkbar, dass die Dichtringlippen auf den Dichtungen oder Membranen angeordnet sind. Sie können dabei so angeordnet sein, dass die Dichtungen oder Membrane unter Vorspannung mit den Dichtringlippen gegen die Teile des Gehäuses drücken, das heißt, die erste Dichtung oder Membran mit ihrer Dichtringlippe gegen den Gehäuseaufsatz und/oder die zweite Dichtung oder Membran mit ihrer Dichtringlippe gegen das Hauptteil.

Diese Ausführungsform hat den Vorteil, dass die Dichtungen oder Membran dadurch etwas steifer sind. Dadurch kann ein noch geringerer Öffnungsdruck der Ventileinrichtung realisiert werden.

Die Erfindung wird im Weiteren anhand der Figuren näher erläutert.

Es zeigt:
- Fig. 1: eine perspektivische Ansicht einer erfindungsgemäßen EinwegVentileinrichtung;
- Fig. 2: eine Explosionsansicht der Einweg-Ventileinrichtung aus Fig. 1;
- Fig. 3: eine geschnittene, perspektivische Ansicht auf die EinwegVentileinrichtung gemäß einer ersten Ausführungsform;
- Fig. 4: eine geschnittene, perspektivische Detailansicht auf das Gehäuse;
- Fig. 5: eine geschnittene, perspektivische Ansicht auf eine Ventileinrichtung gemäß einer zweiten Ausführungsform;
- Fig. 6: eine geschnittene, perspektivische Ansicht auf eine erfindungsgemäße Ventileinrichtung, die in Verbindung mit einer nadellfreien Vorrichtung steht;
- Fig. 7: eine Explosionsansicht der Gesamtvorrichtung aus Fig. 6;
- Fig. 8: eine Detaildarstellung des inneren Gehäuseteils 53 der erfindungsgemäßen Einweg-Ventileinrichtung der Figur 1;
- Fig. 9: eine weitere Ausführungsform einer erfindungsgemäßen EinwegVentileinrichtung;
- Fig. 10: eine Schnittdarstellung der erfindungsgemäßen EinwegVentilvorrichtung der Figur 9;
- Fig. 11: eine Abwandlung der erfindungsgemäßen EinwegVentileinrichtung der Figuren 9 und 10;
- Fig. 12: eine weitere Abwandlung einer erfindungsgemäßen EinwegVentileinrichtung mit einer nadelfreien Vorrichtung und
- Fig. 13: eine Schnittdarstellung der erfindungsgemäßen EinwegVentileinrichtung der Figur 12

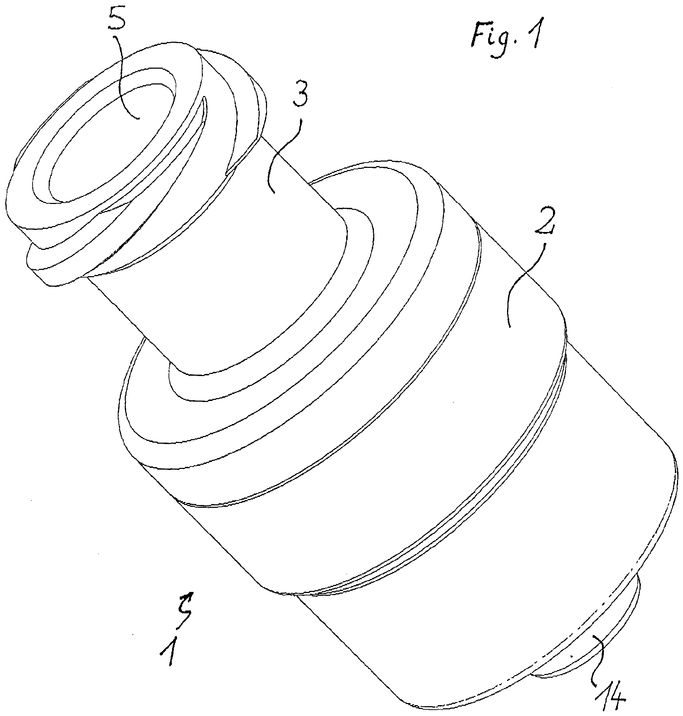

Die in Fig. 1 gezeigte, erfindungsgemäße Einweg-Ventileinrichtung 1 weist insgesamt eine in etwa zylindrische Form auf. Das in etwa zylindrische Gehäuse der Ventileinrichtung 1 umfasst dabei das Hauptteil 2 und den darauf angeordneten Gehäuseaufsatz 3. Der Gehäuseaufsatz 3 umfasst die Zuleitung 5, während das Hauptteil 2 die Ableitung 14 aufweist. Dabei sind die Zu- und Ableitung 5, 14 auf den gegenüberliegenden Stirnflächen des zylindrischen Gehäuses 2, 3 angeordnet. Vorzugsweise, wie der Fig. 1 auch entnehmbar ist, stehen dabei die Zu- und Ableitung senkrecht von den Grundflächen des zylindrischen Gehäuses ab. Denkbar ist jedoch auch, dass sie in einem Winkel oder parallel zu der jeweiligen Stirnfläche abstehen.

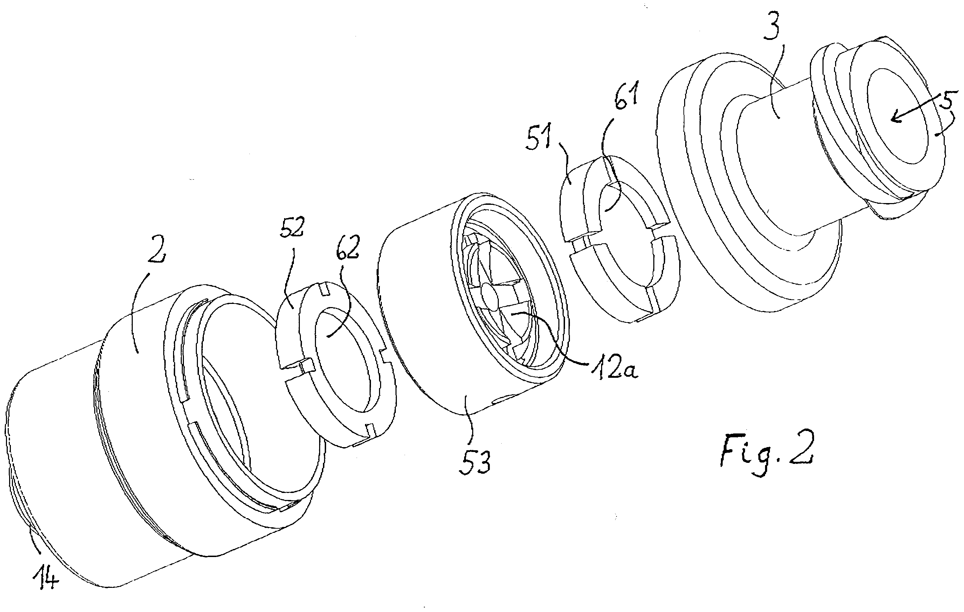

In Fig. 2 ist eine Explosionsansicht der erfindungsgemäßen Ventileinrichtung 1 zu sehen. Dabei sind die einzelnen Teile innerhalb der Ventileinrichtung 1 in ihrer jeweiligen Reihenfolge in Bezug zur Verlaufsrichtung des zu verabreichenden Mittels, welche im Wesentlichen von der Zuleitung 5 in Richtung zu der Ableitung 14 führt, dargestellt.

Innerhalb des Gehäuses 2, 3, das heißt im Wesentlichen innerhalb des Aufnahmebereichs innerhalb des Hauptteiles 2, sind zwei Ventilscheiben 51, 52, sowie dazwischen liegend ein inneres Gehäuseteil 53 angeordnet. Die in Verlaufsrichtung gesehen erste Ventilscheibe 51 weist dabei mindestens eine Dichtung oder Membran 61 auf. In Verlaufsrichtung dahinter angeordnet ist das innere Gehäuseteil 53. Dahinter wiederum ist die zweite Dichtungsscheibe 52, welche mindestens eine Dichtung oder Membran 62 aufweist.

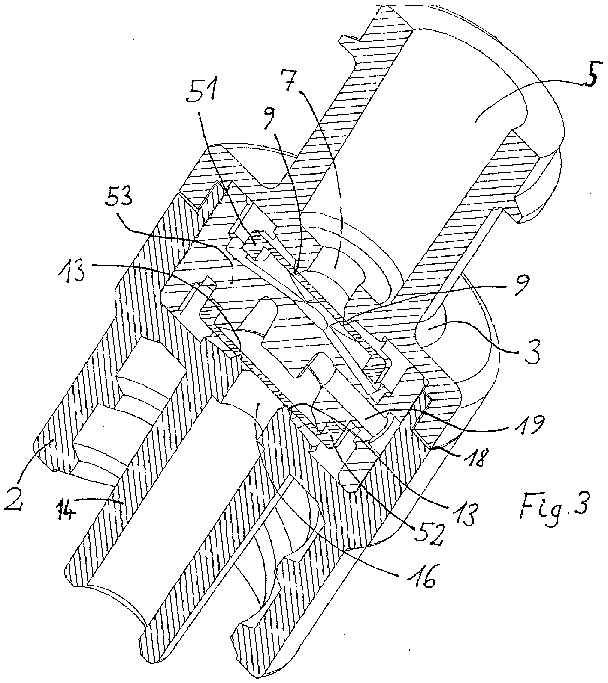

In Fig. 3 ist eine perspektivische Schnittansicht einer Ventileinrichtung gemäß einer ersten Ausführungsform in der zusammengebauten Form dargestellt. Wie zu erkennen ist, ist das innere Gehäuseteil im Verhältnis zu dem Hauptteil 2 und dem Gehäuseaufsatz 3 so ausgebildet, dass die Dichtung 61 der ersten Ventilscheibe 51 zwischen dem inneren Gehäuseteil 53 und dem Gehäuseaufsatz 3 eingepresst ist. Dabei ist die erste Ventilscheibe 51 und/oder die erste Dichtung oder Membran 61 so ausgebildet, dass diese mit Vorspannung an einer Dichtringlippe 9 anliegt, welche sich auf dem Gehäuseaufsatz 3 befindet. Hierdurch kann eine Abdichtung sichergestellt werden, wenn innerhalb der Zuleitung 5 innerhalb des Gehäuseaufsatzes 3 ein Unterdruck besteht. Auch im Normalzustand, d. h. wenn die Ventileinrichtung nicht in Betrieb ist und der Druck überall ausgeglichen ist, kann dadurch eine Abdichtung realisiert werden.

Die Dichtringlippe 9 umgibt dabei vorzugsweise einen Durchbruch 7 innerhalb des Gehäuseaufsatzes 3, welcher Teil der Zuleitung 5 ist. Die Dichtringlippen sind grundsätzlich vorzugsweise in einer Ringform ausgebildet. Diese Ringform umgibt dabei vorzugsweise den Durchbruch 7 der Zuleitung und die Öffnung 16 der Ableitung.

In der gleichen Weise wird eine Abdichtung durch die zweite Ventilscheibe 52 und/oder durch die zweite Dichtung oder Membran 62 und eine zweite Dichtringlippe 13 realisiert, die an dem Hauptteil 2 des Gehäuses angeordnet ist. Die Dichtringlippe 13 umgibt dabei eine Öffnung 16, welche Teil der Ableitung 14 ist. Die Ventilscheibe 52 bzw. die Dichtung 62 sind erneut unter Vorspannung an die Dichtringlippe 13 angepresst. Dabei sitzt erneut die Ventilscheibe 52 - entsprechend der ersten Ventilscheibe 51 - in einem Presssitz zwischen dem inneren Gehäuseteil 53 und dem Hauptteil 2 des Gehäuses. Die zweite Ventilscheibe 52 führt also zu einer Abdichtung, wenn der Druck in allen Bereichen der Ventileinrichtung ausgeglichen ist (d. h. beispielsweise im Nichtbetrieb) und wenn in der Ableitung 14, die sich innerhalb des Hauptteils 2 des Gehäuses befindet, ein Unterdruck besteht.

Der diesbezügliche Referenzdruck wird dabei bei dieser Ausführungsform der Erfindung über einen Durchlassschlitz 18 zwischen den Gehäuseteilen 2 und 3 und einem Kanal 19 in den inneren Ventilteil 53 zur Verfügung gestellt. Dieser Druckraum 12 teilt sich bei der vorliegenden Erfindung in eine Druckkammer 12a unter der Membran 61 und eine Druckkammer 12b unter der Membran 62 auf. Beide Druckkammern 12a, 12b bilden den Druckraum und stehen miteinander und mit der Außenluft wie beschrieben in Verbindung.
Die erste Druckkammer 12a, die sich im Bezug zur Verlaufsrichtung hinter der ersten Ventilscheibe 51 innerhalb des inneren Gehäuseteils 53 befindet, ist zudem so ausgebildet, dass bei einem darin bestehenden Überdruck die Dichtung oder Membran 61 an die Dichtringlippe 9 angedrückt wird und somit schließt. Somit kann beispielsweise ein Überdruck an dem Ableitungsrohr 14, der beispielsweise bei einem vorübergehenden Verschluss der Armvenen des Patienten auftreten kann, nicht dazu führen, dass das zugeführte Mittel wieder durch die Ventileinrichtung entgegen der Verlaufsrichtung zurückfließt.

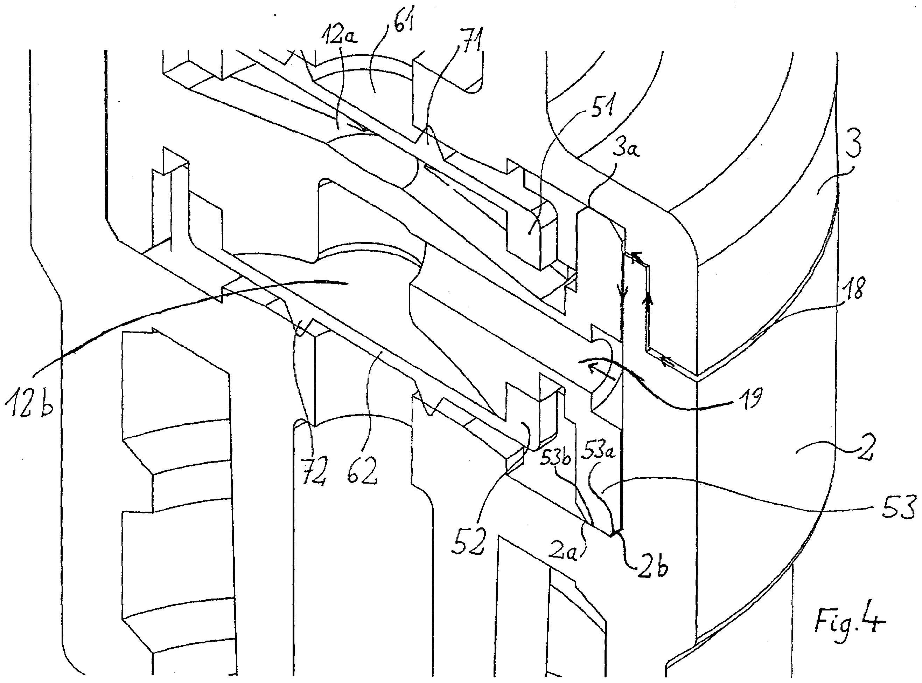

In Fig. 4 ist eine perspektivische Detailansicht des Gehäuses 2, 3 der erfindungsgemäßen Ventileinrichtung gezeigt. Darin wird mittels Pfeilen ein Kanal angedeutet, der sich von einer Gehäuseöffnung 18 zur Druckkammer 12b erstreckt. Diese wird durch die zweite Ventilscheibe 52 auf einer Seite begrenzt. Die Druckkammer 12b ist also gegenüberliegend von derjenigen Seite der Ventilscheibe 52 angeordnet, die in die Richtung der Ableitung 14 gerichtet ist. Der Druckausgleich innerhalb der Druckkammer 12b mit atmosphärischem Druck führt dazu, dass standardmäßig die Druckkammer 12b und somit die entsprechende Seite der Ventilscheibe 52, d. h. der Dichtung 62, mit Atmosphärendruck beaufschlagt ist. Hierdurch wird einerseits ermöglicht, dass die Dichtung 62 durch ein durch die Ventileinrichtung durchfließendes Mittel von der Dichtringlippe 13 angehoben werden kann (was zur Verdrängung der Luft aus der Druckkammer 12b führt), wodurch ein Durchfluss ermöglicht wird, anderseits bei Unterdruck in der Ableitung 14 ein Anpressen der Dichtung 62 an die Dichtringlippe 14 ermöglicht wird (was zum Einsaugen von Luft in die Druckkammer 12b führt).

Die Gehäuseöffnung 18 wird dabei erfindungsgemäß besonders einfach dadurch realisiert, dass die Ultraschallverschweißung zwischen dem Hauptteil 2 und dem Gehäuseaufsatz 3 nicht über den ganzen Umfang des Gehäuses durchgeführt wird, sondern Sektoren ausgelassen werden. In diesem Bereich verbleibt dann ein Abstand zwischen den beiden Gehäuseteile 2 und 3, der diese Gehäuseöffnung 18 realisisert. Dabei ist es besonders bevorzugt, wenn das innere Gehäuseteil 53 mit einer Presspassung zwischen einem kreisringförmigen planen Bereich 2a des Hauptteils 2 und einem ebensolchen planen Bereich 3a des Gehäuseaufsatzes 3 eingepresst ist. Dadurch wird gleichzeitig die Abdichtung der luftführenden Verbindung zwischen der Gehäuseöffnung 18 und dem Druckraum 12 gegenüber den fluidführenden Teilen des Ventiles sichergestellt. Zu diesem Zweck ist es besonders bevorzugt, das innere Gehäuseteil 53 aus einem thermoplastischen Elastomer mit einer Shore-Härte von 70 bis 80A auszuführen.

Weiter kann eine perfekte Zentrierung des inneren Gehäuseteils 53 in dem Hauptteil 2 des Gehäuses und damit auch in dem Gehäuseaufsatz 3 dadurch erzielt werden, dass das innere Gehäuseteil 53 an seinen Außenkanten zwischen Stirn- und Mantelfläche eine Abschrägung 53a aufweist, der eine entsprechende Abschrägung 2b im Übergang zwischen der senkrechten Gehäusewand des Hauptteiles 2 und der kreisringförmigen planen Fläche 2a des Hauptteils 2 entspricht. Auf diese Weise wird beim Zusammenbau der Gehäuseteile 2 und 3 automatisch das vorher zwischen diese Teile eingelegte innere Gehäuseteils 53 optimal zentriert und damit auch ein richtiger Sitz der Ventillippen auf ihren Gegenflächen erreicht.

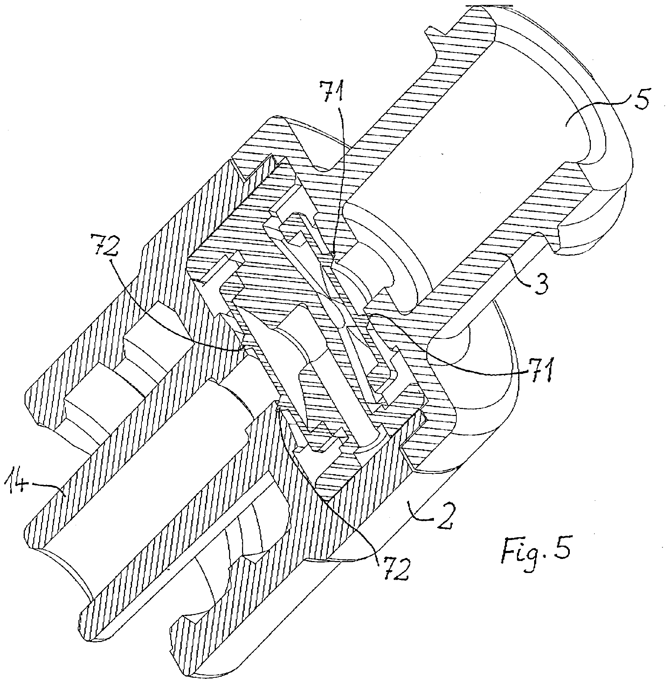

Fig. 5 zeigt eine perspektivische Schnittansicht der erfindungsgemäßen Ventileinrichtung gemäß einer zweiten Ausführungsform. Diese gleicht der Ausführungsform aus Fig. 3, jedoch sind die Dichtringlippen 71, 72 nicht auf den Gehäuseteilen angeordnet, sondern auf den Dichtungen oder Membranen 61, 62. Die Dichtringlippen 71, 72 sind dabei so angeordnet, dass sie jeweils aufgrund der Vorspannung innerhalb der Dichtungen oder Ventilscheiben an die entsprechenden Gehäuseteile 2, 3 angepresst werden. Während also gemäß der ersten Ausführungsform die Dichtringlippen 9, 13 in der durch das Gehäuse 2, 3 definierten Aussparung auf gegenüberliegenden Seiten angeordnet sind und aufeinander gerichtet sind, sind die Dichtringlippen 71, 72 gemäß der zweiten Ausführungsform auf den Dichtungen 61, 62 angeordnet und blicken voneinander weg.

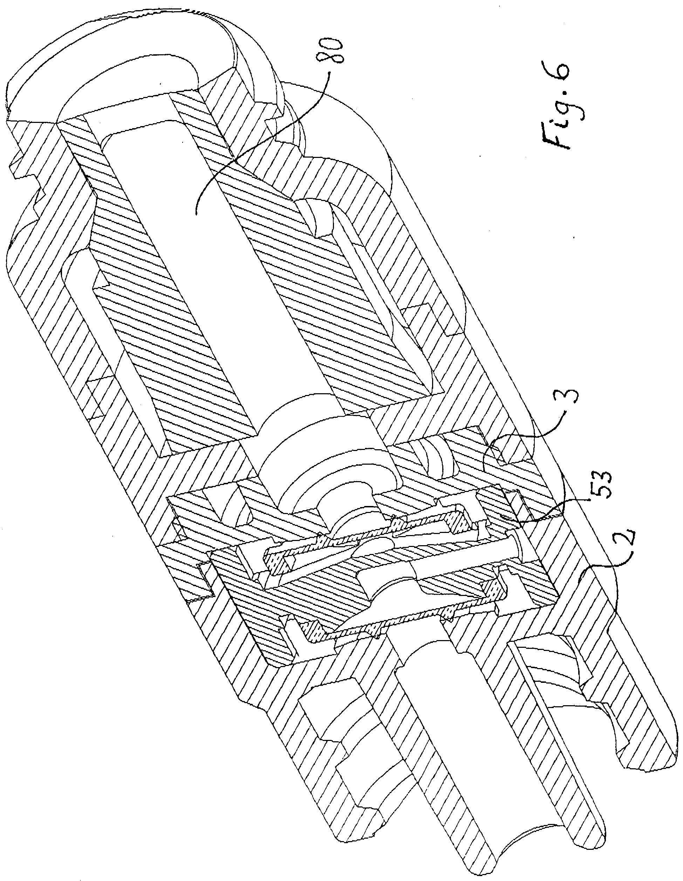

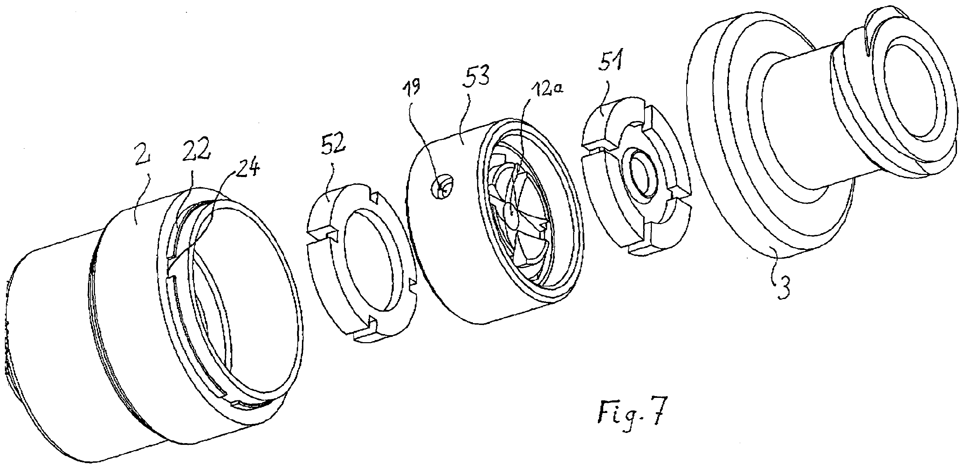

In den Figuren 6 und 7 ist schließlich noch ein Anwendungsbeispiel der erfindungsgemäßen Ventileinrichtung 1 gezeigt. Gemäß diesem Anwendungsbeispiel wird die erfindungsgemäße Ventileinrichtung gemeinsam mit einer standardmäßigen nadelfreien Vorrichtung verwendet. Die nadelfreie Vorrichtung 80 ist dabei an dem Gehäuseaufsatz 3 befestigt. Der Gehäuseaufsatz 3 kann also entsprechend der daran zu befestigenden Vorrichtung angepasst werden. Die erfindungsgemäße Ventileinrichtung ist somit variabel einsetzbar, da bei verschiedenen Anwertdungen die Ventileinrichtung grundsätzlich den gleichen Aufbau aufweisen kann und lediglich der Gehäuseaufsatz 3 angepasst werden muss.

Die Explosionszeichnung der Figur 7 zeigt sehr schön, wie die Gehäuseöffnung 18 realisiert wird, dass heißt wie die Druckkammer 12 über einen Kanal 20 und den Abstand zwischen den Gehäuseteilen 2 und 3 realisiert wird. Wie man in dieser Explosionszeichnung bei der Darstellung des Gehäusehauptteils 2 sieht, sind in einem äußeren Faz des Gehäusehauptteils 2, auf den der Gehäusedeckel 3 aufgesetzt wird, Materialstreifen 22 für die Ultraschallverschweißung der beiden Gehäuseteile 2 und 3 vorgesehen. Diese sind nun nicht ganz umlaufend ausgebildet, sondern decken jeweils Kreissektoren von deutlich weniger als 90 ° ab. Dazwischen sind Unterbrechungen 24 vorgesehen, welche die Luftverbindung der Druckkammer 12 über den Kanal 20 und die zwischen Gehäusehauptteil 2 und Gehäusedeckel 3 gebildete Durchlassöffnung 18 sicherstellen.

Eine weitere Anwendungsmöglichkeit besteht bei Injektionspumpen und Infusionsanwendungen. Die erfindungsgemäße Ventileinrichtung ist nämlich so ausgebildet, dass sie auch bei einem sehr geringen Öffnungsdruck (im allgemeinen zwischen 1,5 und 5 Psi oder zwischen 104 und 345 Millibar) und einem sehr geringen Infusionsvolumen mit einer Infusionsgeschwindigkeit von 0,2 bis 1 mm³ pro Stunde einen konstanten Durchfluss ermöglicht, ein Zurückfließen des Mittels verhindert und einen Abfluss beim Austausch der Zuführungsbehälter beziehungsweise Injektionsvorrichtungen (Pumpen etc.), sowie ein Zuführen von Luft in die Venen des Patienten verhindern kann.

Ein weiteres Anwendungsgebiet der erfindungsgemäßen Ventileinrichtung liegt in den mit Gravitationsdruck arbeitenden Infusionsbestecken. Die erfindungsgemäße Einweg-Ventileinrichtung kann nämlich so ausgebildet sein, dass sie bereits bei einem Druck einer Wassersäule von 15 cm öffnet.

Schließlich ist auch die Anwendung einer peristaltischen Pumpinfusion bei der erfindungsgemäßen Ventileinrichtung möglich. Hierbei ist es jedoch wichtig, dass die Ventileinrichtung direkt hinter der Pumpe in Verlaufsrichtung angeordnet ist, und nicht am distalen Ende (beispielsweise eines zwischen der Pumpe und der Ventileinrichtung angeordneten Schlauchs). Im letztgenannten Fall würde nämlich die benötigte Flüssigkeitssäule hinter der Ventileinrichtung fehlen, die zum korrekten Funktionieren des Anti-Syphon-Ventils notwendig ist. Der Eintritt von Luft in das Infusionsbesteck wäre dann möglich.

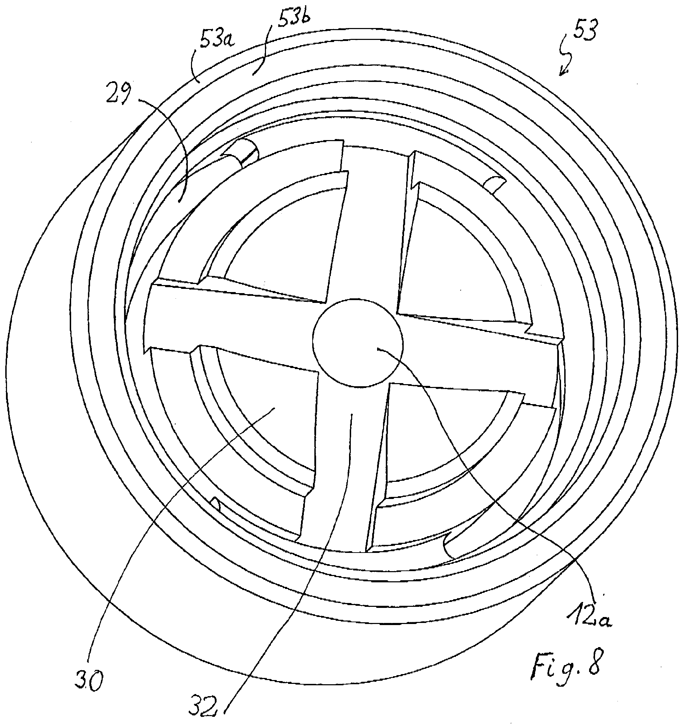

Figur 8 zeigt eine Detaildarstellung des inneren Gehäuseteils 53 der vorbeschrieben erfindungsgemäßen Einweg-Ventileinrichtung 1, von der Einströmseite, d. h. von der Seite der Zuleitung 5 her gesehen, sodass hier in erster Linie die Druckkammer 12a zu erkennen ist. Diese steht in ihrem Randbereich über kreisringabschnitttörmige Durchtrittsöffnungen 29 mit der anderen Druckkammer 12b und über diese mit dem Verbindungskanal 19 wie bei der Beschreibung von Figur 4 näher erläutert mit dem Außenluftdruck in Verbindung.

In Strömungsrichtung wird diese Druckkammer 12a von einer Anlagefläche für die Membran 61 begrenzt. Diese ist aus vier Kreissektoren 30 gebildet, die jeweils in einer konkaven Wölbung nach außen hin ansteigen und so eine Überlastung der Membran vorbeugen, indem sie diese Membran 61 im durchgewölbten Zustand abstützen.

Um die Membran 61 trotzdem noch mit Außenluftdruck beaufschlagen zu können, sind die Sektoren 30 jeweils durch rechteckige Kanäle 32 getrennt, die nach außen hin tiefer werden.

Sehr gut ist in Figur 8 auch die Abschrägung 53a an der Außenkante des inneren Gehäuseteils 53 zu erkennen, die sodann in die planen Fläche 53b übergeht, die für den Presssitz des inneren Gehäuseteils 53 zwischen Gehäuse-Hauptteil 2 und Gehäuseaufsatz 3 erforderlich ist.

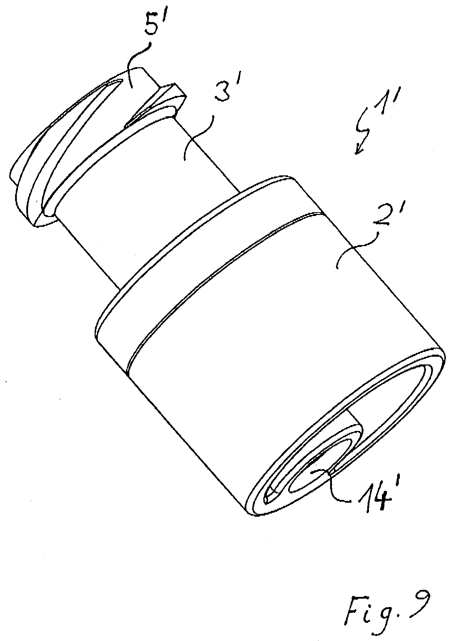

Figur 9 beschreibt eine weitere Ausführungsform der erfindungsgemäßen Einweg-Ventileinrichtung. Diese Konstruktion hat den Nachteil, dass sie nicht rotationssymmetrisch ist, weist dafür aber den Vorteil auf, dass sie keinen Zutritt des äußeren Luftdrucks von der Seite benötigt, sodass sie beispielsweise auch in einen Schlauch eingeschlossen oder mit einer Kunststofffolie umwickelt betrieben werden kann. Auch diese Ausführungsform der vorliegenden Erfindung weist an ihrer Eingangsseite in gleicher Weise wie die in Figur 1 dargestellte Ausführungsform eine in etwa zylindrische Form auf. Auch besteht das in etwa zylindrische Gehäuse der Ventileinrichtung 1' ein Hauptteil 2' und eine darauf angeordnete Gehäuseaufsatz 3' auf, der ebenfalls eine Zuleitung 5' mit einem Außengewinde aufweist. Die Ableitung 14' ist bei dieser Ausführungsform aber exzentrisch angeordnet.

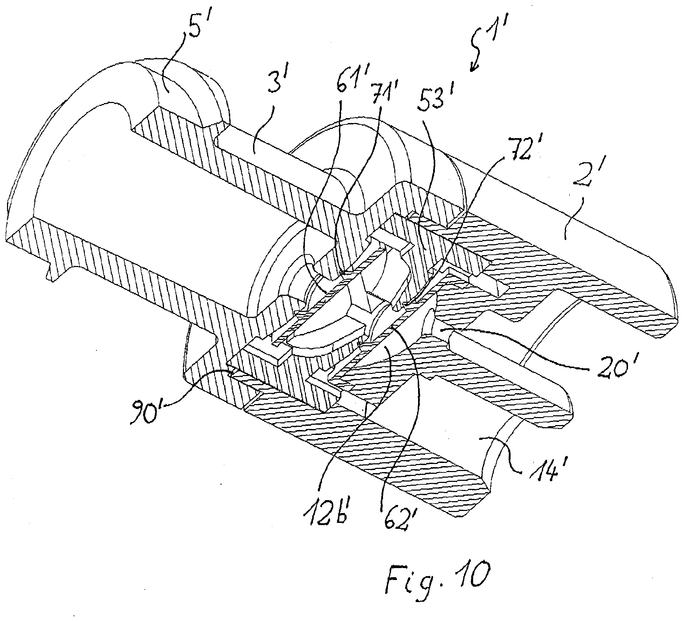

Der Aufbau dieser weiteren Ausführungsform des erfindungsgemäßen Einweg-Ventils 1' wird im folgenden im Einzelnen anhand der Figur 10 näher erläutert. Diese zeigt eine Schnittdarstellung entlang der Rotationsachse des zylindrischen Gehäuses 2'3'.

Wie in Figur 10 erkennbar ist, ist auch hier ein inneres Ventilteil 53' zwischen dem Gehäusehauptteils 2 und dem Gehäuseaufsatz 3' aufgenommen und mit einem Presssitz gehalten. Hier kann das innere Gehäuseteil 53' jedoch deutlich einfacher ausgebildet werden, da es keine seitliche Verbindung mit der äußeren Atmosphäre herstellen muss. Vielmehr ist hier die Fluidableitung 14' exzentrisch angeordnet, und der verbleibende Raum dient dazu, einen von der ableitungsseitigen Stirnseite des Gehäuse-Hauptteils 2' in das Gehäuse führenden, parallel zu der Ableitung 14' angeordneten Druckausgleichskanal 20 zu realisieren, der direkt in die Ableitungsseitige Druckkammer 12b' führt. Im Gegensatz zu der erstbeschrieben Ausführungsform arbeiten hier beide Membranen 61' und 62' in die gleiche Richtung, und nicht gegen einander. Hier ist wieder eine Ausführungsform gezeigt, bei der die entsprechenden Dichtungslippen 71', 72' an den Membranen angebracht sind und auf glatte Ventilsitze an dem Gehäuseaufsatz 3' bzw. dem inneren Gehäuseteil 53' arbeiten.

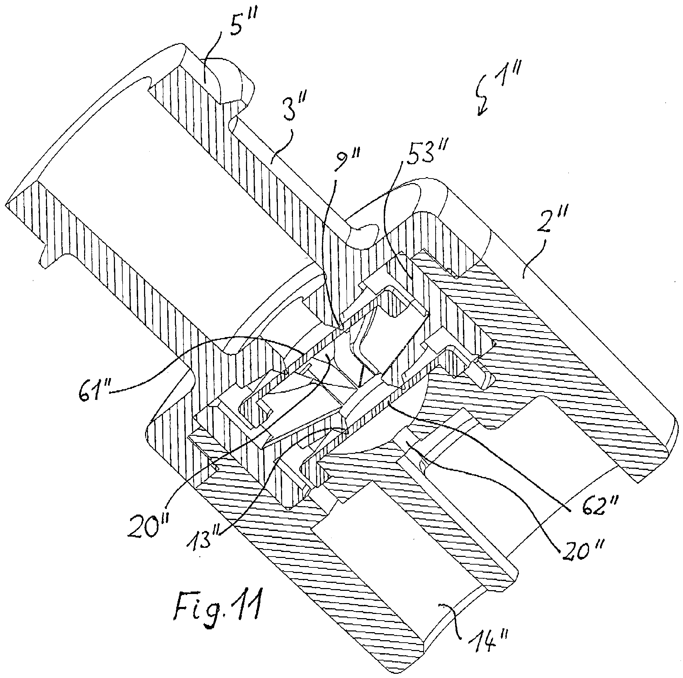

Selbstverständlich kann auch diese Ausführungsform dergestalt abgewandelt werden, dass die Membranen 61', 62' jeweils glatt ausgebildet sind, und entsprechende Lippen 9', 13', auf den Ventilsitzen ausgebildet sind, wie dies in Figur 11 in einer Schnittdarstellung einer entsprechend abgewandelten Ausführungsform der Einweg-Ventileinrichtung 1' dargestellt ist.

Die Figur 11 zeigt darüberhinaus auch eine Darstellung, bei der die Abstützung für die Membran 61 " durch eine größere Anzahl schmalerer kreissektorförmiger Stützelemente 20 " realisiert ist.

Bei der Ausführungsform gemäß den Figuren 10 und 11 wird kein seitlicher Luftzutritt benötigt. Daher können das Gehäuse-Hauptteil 2', 2" und der Gehäuseaufsatz 3', 3" durch eine vollständig rund umlaufende Ultraschallschweißnaht 90', 90" verbunden werden, was die Fertigung natürlich erleichtert.

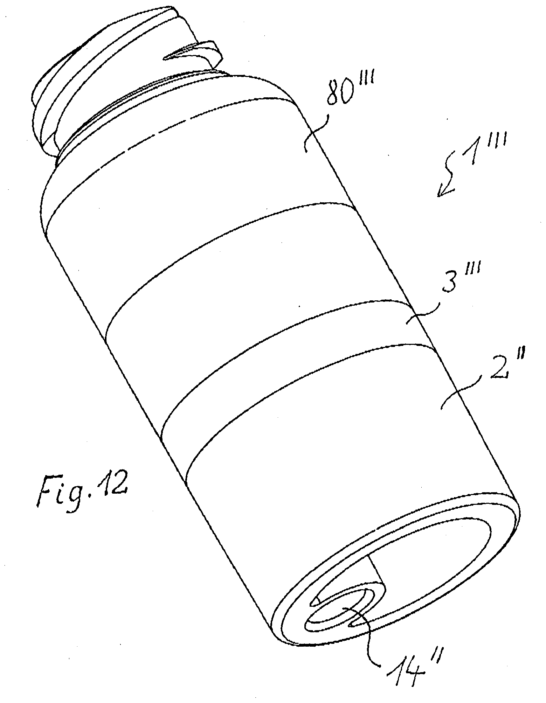

Figur 12 zeigt eine weitere Ausführungsform der erfindungsgemäßen Gestaltung der Figuren 9 bis 11, diesmal jedoch für ein "needlefree device", also eine nadelfreie Vorrichtung.

Auch hier wird wie bei der Ausführungsform gemäß 6 und 7 eine nadelfreie Vorrichtung 80 "' an einen speziell abgewandelten Gehäuseaufsatz 3''' angebracht.

Die Gestaltung der übrigen Teile der erfindungsgemäßen Vorrichtung entspricht der exzentrischen Ausführung der Figuren 9 bis 11, das heißt, dass der Hauptteil 2' des Gehäuses mit der Austrittsöffnung 14' der Gestaltung in Figur 9 bis 11 entspricht.

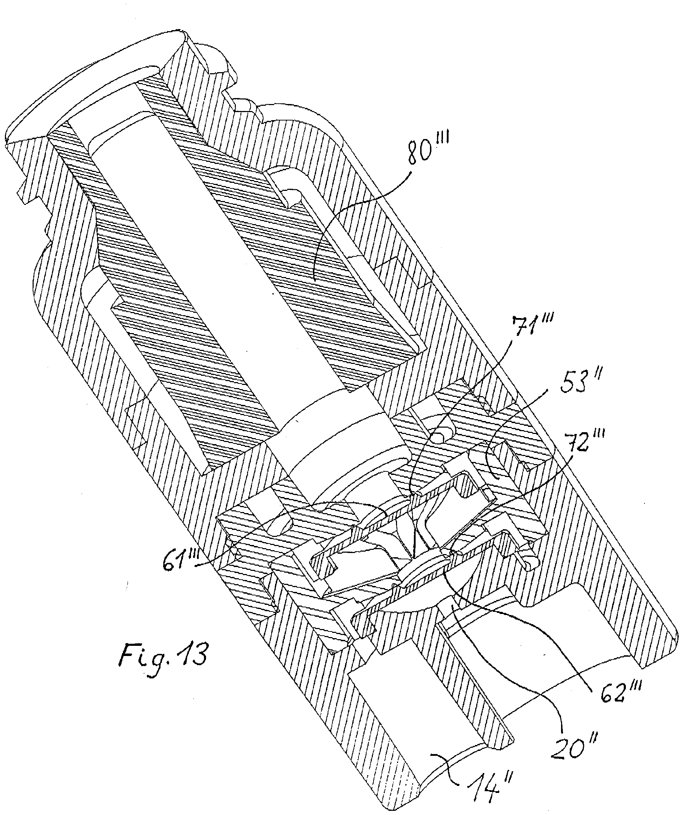

Figur 13 zeigt eine Schnittdarstellung der Ausführungsform der Figur 12, geschnitten entlang der Rotationsachse der zylindrischen Außenform.

In der Schnittdarstellung der Figur 13 ist die Übereinstimmung mit der Schnittdarstellung der Figur 11 im Bereich des Hauptteils 2 " des Gehäuses und des inneren Gehäuseteils 53 " deutlich erkennbar.

Bei dieser Ausführungsform ist jedoch wieder die Gestaltung gewählt worden, dass die Dichtungslippen 71''' und 72''' an den jeweiligen Membranen 61 " bzw. 62''' angeordnet sind, und nicht an dem jeweiligen Ventilsitz, der hier dementsprechend plan ausgeführt ist.

## Patentansprüche

1. Einweg-Ventileinrichtung: (1, 1'; 1"; 1'''), insbesondere Niederdruck-Rückschlagventil, zur Verwendung für ein Infusionsbesteck, mit einem eine Zuleitung (5; 5') und eine Ableitung ('14; 14') aufweisenden Gehäuse (2, 3; 2', 3'; 2", 3"),
wobei die Zu- und Ableitungen (5; 5', 14; 14') mit einem Druckraum in dem Gehäuse (2, 3; 2', 3'; 2", 3") verbunden sind, wobei die Zuleitung (5; 5') auf einer ersten Seite des Gehäuses (2, 3; 2', 3'; 2", 3") und die Ableitung (14; 14') auf einer zweiten, zur ersten gegenüberliegenden Seite des Gehäuses (2, 3; 2', 3'; 2", 3") angeordnet sind, wobei der Druckraum aus zwei miteinander und mittels eines Luftkanals mit Außenluft verbundenen Druckkammern (12a, 12b) aufgebaut ist, und die Einweg-Ventileinrichtung (1, 1'; 1"; 1"') so ausgebildet ist, dass durch einen an der Zuleitung (5; 5') und/oder der Ableitung (14; 14') anstehenden statischen Unterdruck oder einen Überdruck an der Ableitung (14; 14') eine Unterbrechung des Durchflusses erfolgt, **dadurch gekennzeichnet, dass**
in dem Druckraum mindestens zwei die Zuleitung (5; 5') von der Ableitung (14; 14') trennende Dichtungen oder Membranen (61, 62; 61', 62'; 61", 62") vorgesehen sind, und
die zwei Druckkammern (12a, 12b) im Bezug zu der Verlaufsrichtung zwischen der ersten und der zweiten Seite übereinanderliegend im Gehäuse (2, 3; 2', 3'; 2", 3") angeordnet sind, und die zwei Dichtungen oder Membranen (61, 62; 61', 62'; 61", 62") im Bezug zu der Verlaufsrichtung zwischen der ersten und der zweiten Seite übereinanderliegend im Gehäuse (2, 3; 2', 3'; 2", 3") angeordnet sind, wobei das Gehäuse (2, 3; 2', 3'; 2", 3") ein Hauptteil (2; 2'; 2") und einen Gehäuseaufsatz (3; 3'; 3") aufweist,
wobei die Einweg-Ventileinrichtung (1, 1'; 1"; 1"') eine erste und eine zweite Ventilscheibe (51, 52), die jeweils mindestens eine der Dichtungen oder Membranen (61, 62; 61', 62'; 61", 62") umfasst, und ein inneres Ventilteil (53; 53'; 53") aufweist, wobei die Ventilscheiben (51, 52) und das innere Ventilteil (53; 53'; 53") in einer Aussparung zwischen dem Hauptteil (2; 2'; 2") und dem Gehäuseaufsatz (3; 3'; 3") aufgenommen sind und das innere Ventilteil (53; 53'; 53") zwischen den Ventilscheibe (51, 52) angeordnet ist, wobei die erste Ventilscheibe (51) zwischen dem inneren Ventilteil (53; 53'; 53") und dem Gehäuseaufsatz derart eingeklemmt ist, dass die Dichtung oder Membran (61; 61'; 61") unter Vorspannung gegen den Gehäuseaufsatz anliegt, und die zweite Ventilscheibe (52) zwischen dem inneren Gehäuseteil und dem Hauptteil derart eingeklemmt ist, dass die Dichtung oder Membran (62; 62'; 62") unter Vorspannung gegen das Hauptteil anliegt.

2. Einweg-Ventileinrichtung (1, 1'; 1"; 1"') nach einem der vorherigen Ansprüche, **dadurch gekennzeichnet, dass** die zwei Druckkammern (12a, 12b) derart übereinanderliegend in Gehäuse (2, 3; 2', 3'; 2", 3") angeordnet sind, dass sich ihre Grundflächen in einer Aufsicht zumindest teilweise überlappen.

3. Einweg-Ventileinrichtung (1, 1'; 1"; 1''') nach einem der vorherigen Ansprüche, **dadurch gekennzeichnet, dass** mindestens eine der zwei Dichtungen oder Membranen (61, 62; 61', 62'; 61", 62") unter Vorspannung gegen eine entsprechende Dichtringlippe (9, 13; 9', 13') anliegt, die auf dem Gehäuseaufsatz und/oder dem Hauptteil und/oder auf der ersten und/oder zweiten Ventilscheibe (51, 52) angeordnet ist.

4. Einweg-Ventileinrichtung (1, 1'; 1 "; 1''') nach einem der vorherigen Ansprüche, **dadurch gekennzeichnet, dass** mindestens eine der zwei Dichtungen oder Membranen (61, 62; 61', 62'; 61", 62") eine Dichtringlippe (71, 72; 71', 72'; 71''', 72''') aufweist, die unter Vorspannung an dem entsprechenden Teil der Einweg-Ventileinrichtung (1, 1'; 1"; 1''') anliegt.

5. Einweg-Ventileinrichtung (1, 1'; 1"; 1"') nach einem der vorherigen Ansprüche, **dadurch gekennzeichnet, dass** mindestens eine der zwei Ventilscheiben (51, 52) ein Durchgangsloch aufweist.

6. Einweg-Ventiieinrichtung (1, 1'; 1"; 1''') nach einem der vorherigen Ansprüche, **dadurch gekennzeichnet, dass** das Gehäuse (2, 3; 2', 3'; 2", 3") eine zylindrische Form aufweist und die erste und die zweite Seite die Stirnflächen des Zylinders bilden.

7. Einweg-Ventileinrichtung nach einem der Ansprüche 1 bis 4, **dadurch gekennzeichnet, dass** die Zuleitung (5; 5') senkrecht zur ersten Seite des Gehäuses (2, 3; 2', 3'; 2", 3") und/oder die Ableitung (14; 14') senkrecht zur zweiten Seite des Gehäuses (2, 3; 2', 3'; 2", 3") angeordnet ist.

8. Einweg-Ventileinrichtung nach Anspruch 1, **dadurch gekennzeichnet, dass** der Druckraum als zwei über einen Verbindungskanal (40) miteinander verbundene Druckkammern (12a, 12b) ausgebildet ist, und dass der Verbindungskanal einmal oberhalb und einmal unterhalb der Dichtungen oder Membrane (61, 62; 61', 62'; 61", 62") mündet.

9. Einweg-Ventileinrichtung nach Anspruch 1, **dadurch gekennzeichnet, dass** die Zuleitung (5; 5') in eine Vorkammer mündet, in welcher die erste Dichtringlippe (9; 9') mit einer geschlossenen Mulde ausgebildet ist, an der die den Druckraum begrenzende Dichtung oder Membran (61, 62; 61', 62'; 61", 62") anliegt.

10. Einweg-Ventileinrichtung nach Anspruch 1, **dadurch gekennzeichnet, dass** die Ableitung (14; 14') innerhalb der zweiten Dichtringlippe (13; 13') in der Druckkammer des Gehäuses (2, 3; 2', 3'; 2", 3") mündet, an der die den Druckraum begrenzende Dichtung oder Membran (61, 62; 61', 62'; 61", 62") anliegt.

11. Einweg-Ventileinrichtung nach Anspruch 1, **dadurch gekennzeichnet, dass** gegenüber der zweiten Dichtringlippe (13; 13') eine zweite, durch die Dichtung oder Membran (61, 62; 61', 62'; 61", 62") begrenzende Vorkammer vorgesehen ist, welche durch eine Gehäuseöffnung (18) mit Atmosphärendruck beaufschlagt ist.

12. Einweg-Ventileinrichtung nach Anspruch 1 oder 2, **dadurch gekennzeichnet, dass** die Dichtung oder Membran (61, 62; 61', 62'; 61", 62") im Bereich der ersten Dichtringlippe (9; 9') eine Öffnung (16) aufweist, welche zentrisch innerhalb der ersten Dichtringlippe (9) liegend ausgebildet ist, derart, dass der Druckraum gegenüber der Vorkammer (8) abgedichtet ist.

13. Einweg-Ventileinrichtung nach Anspruch 1 oder 2, dass die von der ersten und der zweiten Dichtringlippe (9, 13; 9', 13') eingeschlossenen Flächen kleiner als die jeweils verbleibenden Flächen der anliegenden Dichtung oder Membran (61, 62; 61', 62'; 61", 62") ausgebildet sind.

14. Einweg-Ventileinrichtung nach Anspruch 1 oder 2, **dadurch gekennzeichnet, dass** durch das Verhältnis der eingeschlossenen Flächen innerhalb der Dichtringlippen (9, 13; 9', 13') zu den verbleibenden Flächen der anliegenden Dichtung oder Membran (61, 62; 61', 62'; 61", 62") der Öffnungs- bzw. Schließdruck einstellbar ist.

15. Infusionsbesteck, aufweisend eine Einweg-Ventileinrichtung (1, 1'; 1"; 1"') nach einem der vorherigen Ansprüchen und eine Pumpe, **dadurch gekennzeichnet, dass** die Einweg-Ventileinrichtung (1, 1'; 1"; 1''') in Verlaufsrichtung des zu pumpenden Mittels direkt hinter der Pumpe angeordnet ist.

## Claims

1. One-way valve device (1, 1'; 1 "; 1'''), in particular low-pressure check valve, for use in infusion equipment, with a housing (2, 3; 2', 3'; 2", 3") having an inlet line (5; 5') and an outlet line (14; 14'), the inlet and outlet lines (5; 5', 14; 14') being connected to a pressure space in the housing (2, 3; 2', 3'; 2", 3"), the inlet line (5; 5') being arranged on a first side of the housing (2, 3; 2', 3'; 2", 3"), and the outlet line (14; 14') being arranged on a second side of the housing (2, 3; 2', 3'; 2", 3") opposite the first side, the pressure space being constructed from two interconnected pressure chambers (12a, 12b) connected to outside air by means of an air duct, and the one-way valve device (1, 1'; 1"; 1"') being designed such that the through-flow is interrupted by a static underpressure prevailing on the inlet line (5; 5') and/or the outlet line (14; 14') or by an overpressure on the outlet line (14; 14'), **characterized in that** at least two seals or membranes (61, 62; 61', 62'; 61", 62") separating the inlet line (5; 5') from the outlet line (14; 14') are provided in the pressure space, and the two pressure chambers (12a, 12b) are arranged lying one above the other in the housing (2, 3; 2', 3'; 2", 3") with respect to the flow direction between the first and the second sides, and the two seals or membranes (61, 62; 61', 62'; 61", 62") are arranged lying one above the other in the housing (2, 3; 2', 3'; 2", 3") with respect to the flow direction between the first and second sides, the housing (2, 3; 2', 3'; 2", 3") having a main part (2; 2'; 2") and a housing attachment (3; 3'; 3"), the one-way valve device (1, 1'; 1"; 1''') having a first valve disk (51) and second valve disk (52), which each comprise at least one of the seals or membranes (61, 62; 61', 62'; 61", 62"), and has an inner valve part (53; 53'; 53"), said valve disks (51, 52) and inner valve part (53; 53'; 53") being received in a recess between the main part (2; 2'; 2") and the housing attachment (3; 3'; 3"), and the inner valve part (53; 53'; 53") being arranged between the valve disks (51, 52), the first valve disk (51) being clamped between the inner valve part (53; 53'; 53") and the housing attachment in such a way that the seal or membrane (61; 61'; 61") bears with pretensioning against the housing attachment, and the second valve disk (52) being clamped between the inner housing part and the main part in such a way that the seal or membrane (62; 62'; 62") bears with pretensioning against the main part.

2. One-way valve device (1, 1'; 1"; 1''') according to one of the preceding claims, **characterized in that** the two pressure chambers (12a, 12b) are arranged lying one above the other in the housing (2, 3; 2', 3'; 2", 3") in such a way that their bases at least partially overlap in a plan view.

3. One-way valve device (1, 1'; 1"; 1''') according to one of the preceding claims, **characterized in that** at least one of the two seals or membranes (61, 62; 61', 62'; 61", 62") bears with pretensioning against a corresponding sealing ring lip (9, 13; 9', 13'), which is arranged on the housing attachment and/or the main part and/or on the first and/or second valve disk (51, 52).

4. One-way valve device (1, 1'; 1"; 1''') according to one of the preceding claims, **characterized in that** at least one of the two seals or membranes (61, 62; 61', 62'; 61", 62") has a sealing ring lip (71, 72; 71', 72'; 71''', 72'''), which bears with pretensioning on the corresponding part of the one-way valve device (1, 1'; 1"; 1''').

5. One-way valve device (1, 1'; 1"; 1''') according to one of the preceding claims, **characterized in that** at least one of the two valve disks (51, 52) has a through-hole.

6. One-way valve device (1, 1'; 1"; 1''') according to one of the preceding claims, **characterized in that** the housing (2, 3; 2', 3'; 2", 3") has a cylindrical shape, and the first and second sides form the end faces of the cylinder.

7. One-way valve device according to one of Claims 1 to 4, **characterized in that** the inlet line (5; 5') is arranged perpendicularly with respect to the first side of the housing (2, 3; 2', 3'; 2", 3") and/or the outlet line (14; 14') is arranged perpendicularly with respect to the second side of the housing (2, 3; 2', 3'; 2", 3").

8. One-way valve device according to Claim 1, **characterized in that** the pressure space is designed as two pressure chambers (12a, 12b) connected to each other via a connecting channel (40), and the connecting channel opens out once above and once below the seals or membranes (61, 62; 61', 62'; 61", 62").

9. One-way valve device according to Claim 1, **characterized in that** the inlet line (5; 5') opens into a pre-chamber in which the first sealing ring lip (9; 9') is designed with a closed hollow, on which the seal or membrane (61, 62; 61', 62'; 61", 62") delimiting the pressure space bears.

10. One-way valve device according to Claim 1, **characterized in that** the outlet line (14; 14') opens out inside the second sealing ring lip (13; 13') in the pressure chamber of the housing (2, 3; 2', 3'; 2", 3") on which the seal or membrane (61, 62; 61', 62'; 61", 62") delimiting the pressure space bears.

11. One-way valve device according to Claim 1, **characterized in that** a second pre-chamber, delimited by the seal or membrane (61, 62; 61', 62'; 61", 62"), is provided opposite the second sealing ring lip (13; 13'), which pre-chamber is exposed to atmospheric pressure through a housing opening (18).

12. One-way valve device according to Claim 1 or 2, **characterized in that** the seal or membrane (61, 62; 61', 62'; 61", 62") has, in the area of the first sealing ring lip (9; 9'), an opening (16) which is designed lying centrally inside the first sealing ring lip (9), in such a way that the pressure space is sealed off in relation to the pre-chamber (8).

13. One-way valve device according to Claim 1 or 2, **characterized in that** the surfaces enclosed by the first and second sealing ring lips (9, 13; 9', 13') are designed smaller than the respectively remaining surfaces of the bearing seal or membrane (61, 62; 61', 62'; 61 ", 62").

14. One-way valve device according to Claim 1 or 2, **characterized in that** the opening or closing pressure is adjustable by the ratio of the enclosed surfaces inside the sealing ring lips (9, 13; 9', 13") to the remaining surfaces of the bearing seal or membrane (61, 62; 61', 62'; 61 ", 62").

15. Infusion equipment, having a one-way valve device (1, 1'; 1"; 1''') according to one of the preceding claims and a pump, **characterized in that** the one-way valve device (1, 1'; 1"; 1''') is arranged directly downstream from the pump in the direction of flow of the medium that is to be pumped.

## Revendications

1. Système de valve unidirectionnelle (1, 1'; 1" ; 1'''), en particulier clapet antiretour basse pression pour l'utilisation pour une trousse de perfusion, comprenant un boîtier (2, 3 ; 2', 3' ; 2", 3") présentant une conduite d'alimentation (5 ; 5') et une conduite de sortie (14 ; 14'),
les conduites d'alimentation et de sortie (5 ; 5', 14 ; 14 ') étant connectés à un espace de pression dans le boîtier (2, 3 ; 2', 3'; 2", 3"), la conduite d'alimentation (5 ; 5 ') étant disposée sur un premier côté du boîtier (2, 3 ; 2', 3'; 2", 3") et la conduite de sortie (14 ; 14') étant disposée sur un deuxième côté du boîtier (2, 3 ; 2', 3'; 2", 3") opposé au premier, l'espace de pression étant constitué de deux chambres de pression (12a, 12b) connectées l'une à l'autre et au moyen d'un conduit d'air à l'air extérieur, et
le système de valve unidirectionnelle (1, 1'; 1" ; 1''') étant réalisé de telle sorte qu'il se produise une interruption du débit par une dépression statique au niveau de la conduite d'alimentation (5 ; 5') et/ou de la conduite de sortie (14 ; 14') ou par une surpression au niveau de la conduite de sortie (14 ; 14'),
**caractérisé en ce que**
dans l'espace de pression sont prévus au moins deux joints d'étanchéité ou membranes (61, 62 ; 61', 62' ; 61", 62") séparant la conduite d'alimentation (5 ; 5') de la conduite de sortie (14 ; 14'), et les deux chambres de pression (12a, 12b) sont disposées, par rapport à la direction de propagation entre le premier et le deuxième côté l'une au-dessus de l'autre dans le boîtier (2, 3 ; 2', 3'; 2", 3"), et les deux joints d'étanchéité ou membranes (61, 62 ; 61', 62' ; 61", 62") sont disposés par rapport à la direction de propagation entre le premier et le deuxième côté l'un au-dessus de l'autre dans le boîtier (2, 3 ; 2', 3'; 2", 3"), le boîtier (2, 3 ; 2', 3'; 2", 3") présentant une partie principale (2 ; 2' ; 2") et une coiffe de boîtier (3 ; 3' ; 3"),
le système de valve unidirectionnelle (1, 1'; 1" ; 1''') présentant un premier et un deuxième disque de soupape (51, 52) qui comprend à chaque fois au moins l'un(e) des joints d'étanchéité ou membranes (61, 62 ; 61', 62' ; 61", 62"), et une partie de soupape intérieure (53 ; 53'; 53"), les disques de soupape (51, 52) et la partie de soupape intérieure (53 ; 53'; 53") étant reçus dans un évidement entre la partie principale (2 ; 2' ; 2") et la coiffe de boîtier (3 ; 3' ; 3"), et la partie de soupape intérieure (53 ; 53'; 53") étant disposée entre les disques de soupape (51, 52), le premier disque de soupape (51) étant serré entre la partie de soupape intérieure (53 ; 53'; 53") et la coiffe de boîtier de telle sorte que le joint d'étanchéité ou la membrane (61 ; 61'; 61") s'applique avec précontrainte contre la coiffe de boîtier, et le deuxième disque de soupape (52) étant serré entre la partie de boîtier intérieure et la partie principale de telle sorte que le joint d'étanchéité ou la membrane (62 ; 62'; 62") s'applique avec précontrainte contre la partie principale.

2. Système de valve unidirectionnelle (1, 1'; 1" ; 1''') selon l'une quelconque des revendications précédentes, **caractérisé en ce que** les deux chambres de pression (12a, 12b) sont disposées l'une au-dessus de l'autre dans le boîtier (2, 3 ; 2', 3' ; 2", 3") de telle sorte que leurs surfaces de base se chevauchent au moins en partie en vue de dessus.

3. Système de valve unidirectionnelle (1, 1'; 1" ; 1''') selon l'une quelconque des revendications précédentes, **caractérisé en ce qu'**au moins l'un des deux joints d'étanchéité ou membranes (61, 62 ; 61', 62' ; 61", 62") s'applique avec précontrainte contre une lèvre d'étanchéité annulaire correspondante (9, 13 ; 9', 13') qui est disposée sur la coiffe de boîtier et/ou sur la partie principale et/ou sur le premier et/ou le deuxième disque de soupape (51, 52).

4. Système de valve unidirectionnelle (1, 1'; 1" ; 1''') selon l'une quelconque des revendications précédentes, **caractérisé en ce qu'**au moins l'un(e) des deux joints d'étanchéité ou membranes (61, 62 ; 61', 62' ; 61 ", 62") présente une lèvre d'étanchéité annulaire (71, 72 ; 71', 72'; 71''', 72''') qui s'applique avec précontrainte contre la partie correspondante du système de valve unidirectionnelle (1, 1' ; 1" ; 1''').

5. Système de valve unidirectionnelle (1, 1'; 1" ; 1''') selon l'une quelconque des revendications précédentes, **caractérisé en ce qu'**au moins l'un des deux disques de soupape (51, 52) présente un trou traversant.

6. Système de valve unidirectionnelle (1, 1' ; 1" ; 1''') selon l'une quelconque des revendications précédentes, **caractérisé en ce que** le boîtier (2, 3 ; 2', 3' ; 2", 3") présente une forme cylindrique et le premier et le deuxième côté forment les surfaces frontales du cylindre.

7. Système de valve unidirectionnelle selon l'une quelconque des revendications 1 à 4, **caractérisé en ce que** la conduite d'alimentation (5 ; 5') est disposée perpendiculairement au premier côté du boîtier (2, 3 ; 2', 3' ; 2", 3") et/ou la conduite d'évacuation (14 ; 14') est disposée perpendiculairement au deuxième côté du boîtier (2, 3 ; 2', 3' ; 2", 3").

8. Système de valve unidirectionnelle selon la revendication 1, **caractérisé en ce que** l'espace de pression est réalisé sous forme de deux chambres de pression (12a, 12b) connectées l'une à l'autre par le biais d'un conduit de liaison (40) et **en ce que** le conduit de liaison débouche une fois au-dessus et une fois en dessous des joints d'étanchéité ou membranes (61, 62 ; 61', 62' ; 61", 62").

9. Système de valve unidirectionnelle selon la revendication 1, **caractérisé en ce que** la conduite d'alimentation (5 ; 5') débouche dans une préchambre dans laquelle est réalisée la première lèvre d'étanchéité annulaire (9 ; 9'), avec un creux fermé au niveau duquel s'applique le joint d'étanchéité ou la membrane (61, 62 ; 61', 62' ; 61 ", 62") limitant l'espace de pression.

10. Système de valve unidirectionnelle selon la revendication 1, **caractérisé en ce que** la conduite d'évacuation (14 ; 14') débouche à l'intérieur de la deuxième lèvre d'étanchéité annulaire (13 ; 13') dans la chambre de pression du boîtier (2, 3 ; 2', 3' ; 2", 3"), au niveau de laquelle s'applique le joint d'étanchéité ou la membrane (61, 62 ; 61', 62' ; 61", 62") limitant l'espace de pression.

11. Système de valve unidirectionnelle selon la revendication 1, **caractérisé en ce qu'**en face de la deuxième lèvre d'étanchéité annulaire (13 ; 13'), il est prévu une deuxième préchambre limitée par le joint d'étanchéité ou la membrane (61, 62 ; 61', 62' ; 61 ", 62"), laquelle est sollicitée par une ouverture de boîtier (18) avec la pression atmosphérique.

12. Système de valve unidirectionnelle selon la revendication 1 ou 2, **caractérisé en ce que** le joint d'étanchéité ou la membrane (61, 62 ; 61', 62' ; 61", 62") présente, dans la région de la première lèvre d'étanchéité annulaire (9 ; 9'), une ouverture (16) qui est réalisée de manière à être située centralement à l'intérieur de la première lèvre d'étanchéité annulaire (9) de telle sorte que l'espace de pression soit étanchéifié par rapport à la préchambre (8).

13. Système de valve unidirectionnelle selon la revendication 1 ou 2, **caractérisé en ce que** les surfaces renfermées par la première et la deuxième lèvre d'étanchéité annulaires (9, 13 ; 9', 13') sont plus petites que les surfaces respectivement restantes du joint d'étanchéité ou de la membrane (61, 62 ; 61', 62' ; 61", 62") en appui.

14. Système de valve unidirectionnelle selon la revendication 1 ou 2, **caractérisé en ce que** la pression d'ouverture ou de fermeture peut être ajustée par le rapport des surfaces incluses à l'intérieur des lèvres d'étanchéité annulaires (9, 13 ; 9', 13') aux surfaces restantes du joint d'étanchéité ou de la membrane (61, 62 ; 61', 62' ; 61", 62") en appui.

15. Trousse de perfusion, présentant un système de valve unidirectionnelle (1, 1' ; 1" ; 1''') selon l'une quelconque des revendications précédentes et une pompe, **caractérisée en ce que** le système de valve unidirectionnelle (1, 1' ; 1" ; 1''') est disposé directement derrière la pompe dans la direction de propagation du milieu à pomper.
